# EUROPEAN PATENT APPLICATION

(11) **EP 3 633 682 A1**
(43) Date of publication of application: **08.04.2020**
(21) Application number: 19189642.2
(22) Date of filing: 01.08.2019
(51) Int. Cl.: G16B 40/30, G06T 7/00

(54) **CHROMOSOME ABNORMALITY DETECTING MODEL, DETECTING SYSTEM THEREOF, AND METHOD FOR DETECTING CHROMOSOME ABNORMALITY**

(30) Priority: 05.10.2018 TW 107135294; 21.02.2019 TW 108105869
(71) Applicant: China Medical University Hospital, Taichung City 404 (TW)
(72) Inventor: TSAI, Fuu-Jen, 406 Taichung City (TW); HUANG, Tzung-Chi, 404 Taichung City (TW); LIAO, Ken Ying-Kai, Taichung City (TW); YU, Jiaxin, 408 Taichung City (TW); HSIEH, Po-Hsin, 709 Tainan City (TW)
(74) Representative: dompatent von Kreisler Selting Werner - Partnerschaft von Patent- und Rechtsanwälten mbB

(57) **Abstract**

A chromosome abnormality detecting system includes an image capturing unit and a non-transitory machine readable medium. The image capturing unit is for obtaining a target metaphase chromosomes image of a subject. The non-transitory machine readable medium is signal connected to the image capturing unit and stores a program which, when executed by at least one processing unit, determines whether the subject has a chromosome abnormality when executed by a processing unit. The program includes a reference database obtaining module, a reference image transforming module, a reference preliminary classifying module, a reference feature selecting module, a training module, a target image transforming module, a target preliminary classifying module, a target feature selecting module and a comparing module.

## Description

### BACKGROUND

### Technical Field

The present disclosure relates to a medical information analysis model, system and method thereof. More particularly, the present disclosure relates to a chromosome abnormality detecting model, a chromosome abnormality detecting system, and a method for detecting chromosome abnormality.

### Description of Related Art

Detections of the chromosomal abnormality are used for genetic disease screening or for cancer cell mutation detection, such as blood cancer and lymphoma. The genetic disease screening is mainly for pregnant women to receive relevant tests during pregnancy. Because the chromosomes of the fetus include the chromosome of the sperm cell of the father and the chromosome of the egg cell of the mother after meiosis, it is possible to generate a chromosomal mutation in the embryo. Therefore, it is necessary to confirm the health status of the fetus by detecting the abnormality of the fetal chromosome.

The chromosome abnormality can be generally classified into an abnormal number of chromosomes, a chromosome structural abnormality or a chromosome mosaicism. The abnormal number of chromosomes result in nondisjunction of the chromosome during the meiosis of the germ cells, thereby the number of the chromosome of the sperm cell or the egg cell will be abnormal. After the conception, the number of chromosomes of the fetus will be haploid or polyploid, and a congenital anomaly fetus is born. Common abnormal number of chromosomes include trisomy 21 (Down's disease), trisomy 18 (Edd's disease), and single chromosome X (Turner's disease). The chromosome structural abnormality is caused by one or more defects and abnormal combinations of chromosome structures. Common chromosome mosaicisms includes the chromosome mosaicism of Down's syndrome (46, XX/47, XX, +21), and the chromosome mosaicism of Turner's disease (45, X/46, XX; 45, X/46, XY or 45, X/46, X, i(Xq)). Generally, the symptoms of the chromosome mosaicism having some normal chromosome cells usually lighter than the chromosome mosaicism without normal chromosome cells.

The conventional method for detecting chromosomal abnormality is to photograph the metaphase chromosomes image, and the metaphase chromosomes image is artificially arranged by the inspector to obtain a chromosome karyotype image. Then using the chromosome karyotype image to determine whether the subject has abnormal number of chromosomes, such as being haploid or polyploid, and whether the subject has the chromosome structural abnormality, such as a chromosome deletion, a ring chromosome, a chromosome translocation or a chromosome inversion. The determination results of the detection of the chromosomal abnormality are extremely different among different inspectors, and the process is complicated and time consuming. Therefore, it is necessary to improve the conventional techniques to improve the accuracy of the diagnosis of breast tumor types by using breast ultrasound image, reduce the discomfort caused by other invasive examinations, and reduce the spread of cancer cells that may be caused by the examination. Therefore, how to develop a chromosome abnormality detecting system with high accuracy and rapid detection is a technical issue with commercial value.

### SUMMARY

According to one aspect of the present disclosure, a chromosome abnormality detecting model includes following establishing steps. A reference database is obtained, wherein the reference database includes a plurality of reference metaphase chromosomes images. An image transforming step is performed, wherein the image transforming step is for arranging 23 pairs of chromosomes of each of the reference metaphase chromosomes images by an unsupervised learning classifier to obtain a plurality of reference chromosome karyotype images. A preliminary classifying step is performed, wherein the preliminary classifying step is for classifying the reference chromosome karyotype images according to a number of the chromosomes. When the number of chromosomes is 46, the reference chromosome karyotype image is classified into that a reference subject has a normal number of chromosomes. When the number of chromosomes is greater than or less than 46, the reference chromosome karyotype image is classified into that the reference subject an abnormal number of chromosomes. A feature selecting step is performed, wherein the feature selecting step is for analyzing the reference chromosome karyotype images by using a feature selecting module to obtain at least one image eigenvalue. A training step is performed, wherein the training step is for achieving a convergence of the image eigenvalue by using a convolutional neural network learning classifier to obtain the chromosome abnormality detecting model. The chromosome abnormality detecting model is used to determine whether a subject has a chromosome abnormality.

In one example, the unsupervised learning classifier can be a Generative Adversarial Network (GAN).

In one example, the at least one image eigenvalue can include a chromosome size, a chromosome location or a chromosome shape.

In one example, the convolutional neural network learning classifier can be an Inception-ResNet-v2 convolutional neural network or an Inception V3 convolutional neural network.

In one example, the chromosome abnormality can include an abnormal number of chromosomes, a chromosome structural abnormality or a chromosome mosaicism.

According to another aspect of the present disclosure, a method for detecting chromosome abnormality includes following steps. The chromosome abnormality detecting model of the aforementioned aspect is provided. A target metaphase chromosomes image of a subject is provided. A target chromosome karyotype image is obtained by arranging 23 pairs of chromosomes of the target metaphase chromosomes image by the unsupervised learning classifier. The chromosome abnormality detecting model is used to analyze the target chromosome karyotype image to determine whether the subject has a chromosome abnormality.

In one example, the chromosome abnormality can include an abnormal number of chromosomes, a chromosome structural abnormality or a chromosome mosaicism.

In one example, the abnormal number of chromosomes can include target chromosomes of the subject being a haploid or a polyploid.

In one example, the chromosome structural abnormality can include target chromosomes of the subject being a chromosome deletion, a ring chromosome, a chromosome translocation, a chromosome inversion or a chromosome duplication.

According to still another aspect of the present disclosure, a chromosome abnormality detecting system includes an image capturing unit and a non-transitory machine readable medium. The image capturing unit is for obtaining a target metaphase chromosomes image of a subject. The non-transitory machine readable medium is signal connected to the image capturing unit and stores a program which, when executed by at least one processing unit, determines whether the subject has a chromosome abnormality. The program includes a reference database obtaining module, a reference image transforming module, a reference preliminary classifying module, a reference feature selecting module, a training module, a target image transforming module, a target preliminary classifying module, a target feature selecting module and a comparing module. The reference database obtaining module is for obtaining a reference database, wherein the reference database includes a plurality of reference metaphase chromosomes images. The first reference image transforming module is for arranging 23 pairs of chromosomes of each of the reference metaphase chromosomes images by an unsupervised learning classifier to obtain a plurality of reference chromosome karyotype images. The reference preliminary classifying module is for classifying the reference chromosome karyotype images according to a number of the reference chromosomes. When the number of the reference chromosomes is 46, the reference chromosome karyotype image is classified into that a reference subject has a normal number of chromosomes. When the number of the reference chromosomes is greater than or less than 46, the reference chromosome karyotype image is classified into that the reference subject has an abnormal number of chromosomes. The reference feature selecting module is for analyzing the reference chromosome karyotype images to obtain at least one reference image eigenvalue. The training module is for achieving a convergence of the reference image eigenvalue by using a convolutional neural network learning classifier to obtain a chromosome abnormality detecting model. The target image transforming module is for arranging 23 pairs of chromosomes of the target metaphase chromosomes image by the unsupervised learning classifier to obtain a target chromosome karyotype image. The target preliminary classifying module is for classifying the target chromosome karyotype images according to a number of the target chromosomes. When the number of the target chromosomes is 46, the target chromosome karyotype image is classified into that the subject has the normal number of chromosomes. When the number of the target chromosomes is greater than or less than 46, the target chromosome karyotype image is classified into that the subject has the abnormal number of chromosomes. The target feature selecting module is for analyzing the target chromosome karyotype images to obtain at least one target image eigenvalue. The comparing module is for analyzing the at least one target image eigenvalue by the chromosome abnormality detecting model to obtain a target image eigenvalue weight data to determine whether the subject has a chromosome structural abnormality or a chromosome mosaicism.

In one example, the unsupervised learning classifier can be a Generative Adversarial Network (GAN).

In one example, the at least one reference image eigenvalue can include a chromosome size, a chromosome location or a chromosome shape, and the at least one target image eigenvalue comprises a chromosome size, a chromosome location or a chromosome shape.

In one example, the convolutional neural network learning classifier can be an Inception-ResNet-v2 convolutional neural network or an Inception V3 convolutional neural network.

In one example, the program of the non-transitory machine readable medium can further include an assessing module for calculating a value-at-risk of the subject having the chromosome abnormality according to the target image eigenvalue weight data.

### BRIEF DESCRIPTION OF THE DRAWINGS

The present disclosure can be more fully understood by reading the following detailed description of the embodiment, with reference made to the accompanying drawings as follows:
Fig. 1 is a flowchart of establishing steps of a chromosome abnormality detecting model according to one embodiment of the present disclosure.
Fig. 2 is a flowchart of a method for detecting chromosome abnormality according to another embodiment of the present disclosure.
Fig. 3 is a block diagram of a chromosome abnormality detecting system according to still another embodiment of the present disclosure.
Fig. 4 shows a result of a transformation of a target metaphase chromosomes image into a target chromosome karyotype image.
Fig. 5 is a structural diagram of a convolutional neural network learning classifier of a chromosome abnormality detecting model according to one example of one embodiment of the present disclosure.
Fig. 6 is a structural diagram of a convolutional neural network learning classifier of a chromosome abnormality detecting model according to another example of one embodiment of the present disclosure.
Fig. 7 is a confusion matrix of the chromosome abnormality detecting model of the present disclosure used to determine a chromosome abnormality of a subject.

### DETAILED DESCRIPTION

Reference will now be made in detail to the present embodiments of the present disclosure, examples of which are illustrated in the accompanying drawings.

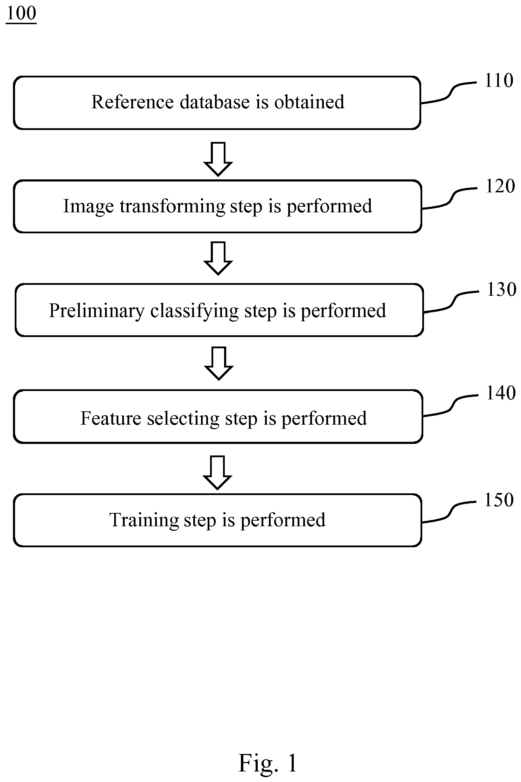

Please refer to Fig. 1, which is a flowchart of establishing steps of a chromosome abnormality detecting model 100 according to one embodiment of the present disclosure. The establishing steps of the chromosome abnormality detecting model 100 includes Step 110, Step 120, Step 130, Step 140 and Step 150. The established chromosome abnormality detecting model can be used to determine whether a subject has an abnormal number of chromosomes, a chromosome structural abnormality or a chromosome mosaicism.

In Step 110, a reference database is obtained, wherein the reference database includes a plurality of reference metaphase chromosomes images. In non-dividing cells, the chromatin is distributed in the nucleus from 30 nm to 300 nm. When the cells enter the mitosis phase, the chromosomes begin to be closely arranged. When the cell is in the metaphase, the nuclear membrane of the cell disappears completely and the spindle begins to become clear. The centromeres on each chromosome are attached to the spindle fiber (or astral fiber), and the centromere is moved up and down by the two-pole tension. The final two-pole tension is balanced, and the centromere is arranged on the equatorial plate in the center of the cell, which is the time that the resolution of the chromosome reaches the highest. Therefore, before obtaining the reference metaphase chromosomes image, the reference subjects are administrated hormone, thereby the cells of the reference subjects are in the metaphase. Then, the specific cells of the reference subjects are extracted, and the reference metaphase chromosomes images are obtained by staining and microscopic observation.

In Step 120, an image transforming step is performed. The image transforming step is for arranging 23 pairs of chromosomes of each of the reference metaphase chromosomes images by an unsupervised learning classifier to obtain a plurality of reference chromosome karyotype images. The reference chromosome karyotype images are obtained by analyzing, comparing, sorting, and numbering the reference metaphase chromosomes images according to features of the chromosomes including the length of the chromosome, the location of the centromere, the ratio of the longer arm and the shorter arm, and the presence or absence of the satellite. The unsupervised learning classifier can be a Generative Adversarial Network (GAN).

In Step 130, a preliminary classifying step is performed. The preliminary classifying step is for classifying the reference chromosome karyotype images according to a number of the chromosomes. When the number of chromosomes is 46, the reference chromosome karyotype image is classified into that a reference subject has a normal number of chromosomes. When the number of chromosomes is greater than or less than 46, the reference chromosome karyotype image is classified into that the reference subject has an abnormal number of chromosomes.

In Step 140, a feature selecting step is performed. The feature selecting step is for analyzing the reference chromosome karyotype images by using a feature selecting module to obtain at least one image eigenvalue. The least one image eigenvalue includes a chromosome size, a chromosome location or a chromosome shape.

In Step 150, a training step is performed. The training step is for achieving a convergence of the image eigenvalue by using a convolutional neural network learning classifier to obtain the chromosome abnormality detecting model. The convolutional neural network learning classifier can be an Inception-ResNet-v2 convolutional neural network or an Inception V3 convolutional neural network.

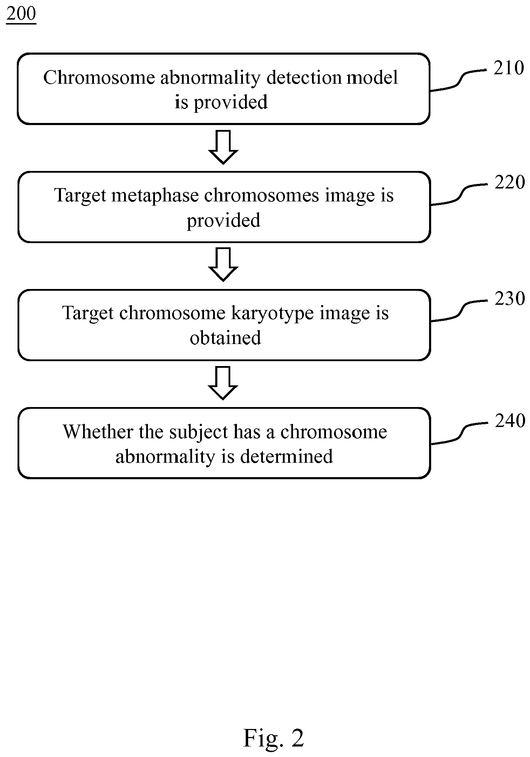

Please refer to Fig. 2, which is a flowchart of a method for detecting chromosome abnormality 200 according to another embodiment of the present disclosure. The method for detecting chromosome abnormality 200 includes Step 210, Step 220, Step 230 and Step 240.

In Step 210, the chromosome abnormality detecting model is provided, wherein the chromosome abnormality detecting model is established by the aforementioned Steps 110 to 150.

In Step 220, a target metaphase chromosomes image of a subject is provided. Before obtaining the target metaphase chromosomes image, the subjects is administrated hormone, thereby the cells of the subject are in the metaphase. Then, the specific cells of the subject are extracted, and the target metaphase chromosomes image is obtained by staining and microscopic observation.

In Step 230, a target chromosome karyotype image is obtained by arranging 23 pairs of chromosomes of the target metaphase chromosomes image by the unsupervised learning classifier. The target chromosome karyotype image is obtained by analyzing, comparing, sorting, and numbering the target metaphase chromosomes image according to features of the chromosomes including the length of the chromosome, the location of the centromere, the ratio of the longer arm and the shorter arm, and the presence or absence of the satellite. The unsupervised learning classifier can be a Generative Adversarial Network (GAN).

In Step 240, the chromosome abnormality detecting model is used to analyze the target chromosome karyotype image to determine whether the subject has the chromosome abnormality. The chromosome abnormality can include an abnormal number of chromosomes, a chromosome structural abnormality or a chromosome mosaicism. The abnormal number of chromosomes can include target chromosomes of the subject being a haploid or a polyploid. The chromosome structural abnormality can include target chromosomes of the subject being a chromosome deletion, a ring chromosome, a chromosome translocation, a chromosome inversion or a chromosome duplication.

Therefore, the chromosome abnormality detecting model and the method for detecting chromosome abnormality of the present disclosure can effectively reduce the error caused by the subjective consciousness of different inspectors in the detection of the chromosome abnormality by automatically transforming the target metaphase chromosomes image into the target chromosome karyotype image, and using the feature selecting module to analyze the target chromosome karyotype image to obtain at least one image eigenvalue. Furthermore, the chromosome abnormality detecting model with deep neural network learning function can not only effectively improve the accuracy and sensitivity of the detection of the chromosome abnormality, but also greatly shorten the determination time of the chromosome abnormality. Accordingly, the chromosome abnormality detecting model and the method for detecting chromosome abnormality of the present disclosure are more efficient in detecting the chromosome abnormality.

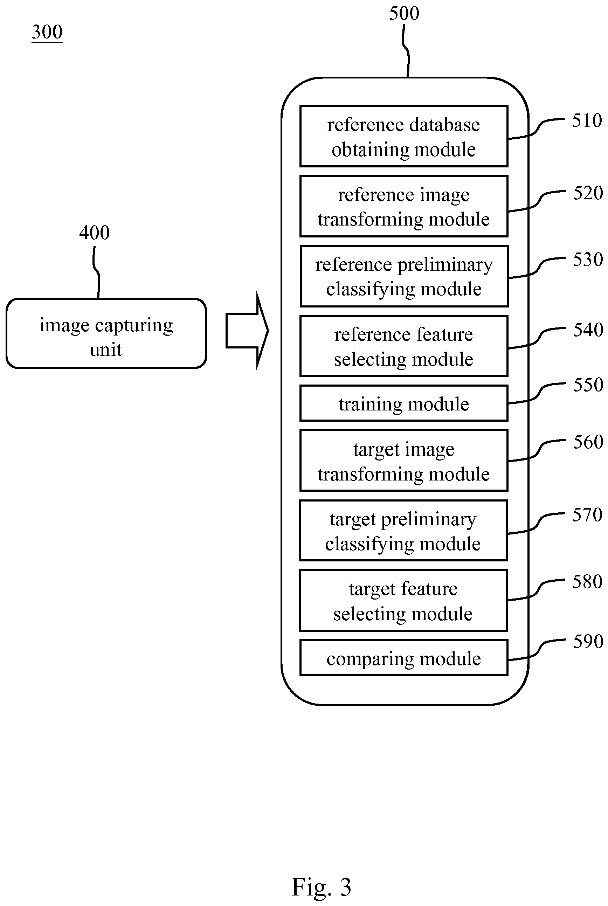

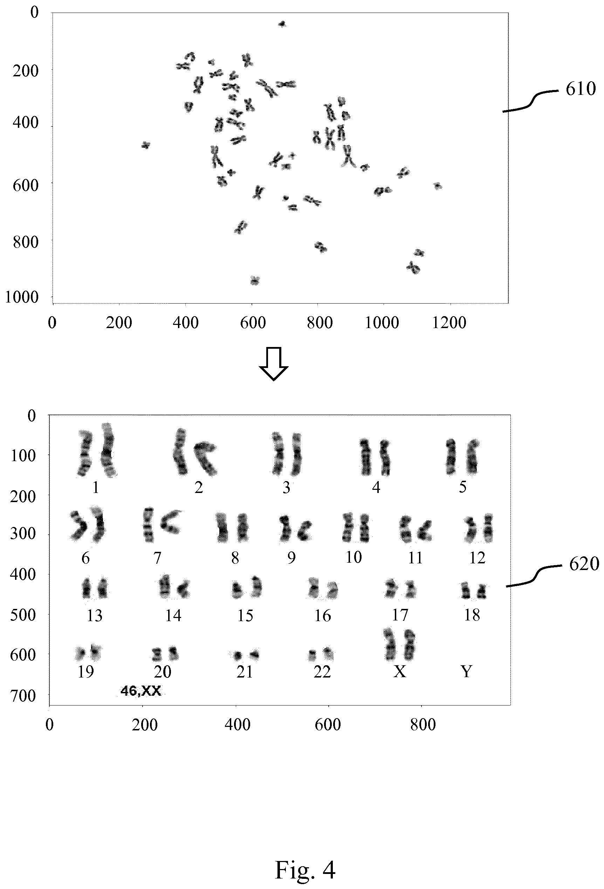

Please refer to Figs. 3 and 4, Fig. 3 is a block diagram of a chromosome abnormality detecting system 300 according to still another embodiment of the present disclosure, and Fig. 4 shows a result of a transformation of a target metaphase chromosomes image 610 into a target chromosome karyotype image 620. The chromosome abnormality detecting system 300 includes an image capturing unit 400 and a non-transitory machine readable medium 500. The chromosome abnormality detecting system 300 can be used to determine whether the subject has the abnormal number of chromosomes, the chromosome structural abnormality or the chromosome mosaicism.

The image capturing unit 400 is for obtaining the target metaphase chromosomes image 610 of the subject. The image capturing unit 400 can be an image capturing device cooperated with a microscope for taking a chromosome image observed by the microscope.

The non-transitory machine readable medium 500 is signal connected to the image capturing unit 400 and stores a program which, when executed by at least one processing unit, determines whether the subject has the chromosome abnormality. The program includes a reference database obtaining module 510, a reference image transforming module 520, a reference preliminary classifying module 530, a reference feature selecting module 540, a training module 550, a target image transforming module 560, a target preliminary classifying module 570, a target feature selecting module 580 and a comparing module 590.

The reference database obtaining module 510 is for obtaining a reference database, wherein the reference database includes a plurality of reference metaphase chromosomes images.

The first reference image transforming module 520 is for arranging 23 pairs of chromosomes of each of the reference metaphase chromosomes images by an unsupervised learning classifier to obtain a plurality of reference chromosome karyotype images. The unsupervised learning classifier can be the Generative Adversarial Network (GAN).

The reference preliminary classifying module 530 is for classifying the reference chromosome karyotype images according to a number of the reference chromosomes. When the number of the reference chromosomes is 46, the reference chromosome karyotype image is classified into that the reference subject has the normal number of chromosomes. When the number of the reference chromosomes is greater than or less than 46, the reference chromosome karyotype image is classified into that the reference subject has the abnormal number of chromosomes. Preferably, the abnormal number of chromosomes can include reference chromosomes being a haploid or a polyploid.

The reference feature selecting module 540 is for analyzing the reference chromosome karyotype images to obtain at least one reference image eigenvalue. The at least one reference image eigenvalue can include a chromosome size, a chromosome location or a chromosome shape.

The training module 550 is for achieving a convergence of the reference image eigenvalue by using a convolutional neural network learning classifier to obtain the chromosome abnormality detecting model. The convolutional neural network learning classifier can be an Inception-ResNet-v2 convolutional neural network or an Inception V3 convolutional neural network.

The target image transforming module 560 is for arranging 23 pairs of chromosomes of the target metaphase chromosomes image 610 by the unsupervised learning classifier to obtain a target chromosome karyotype image 620. The unsupervised learning classifier can be the Generative Adversarial Network (GAN).

The target preliminary classifying module 570 is for classifying the target chromosome karyotype images according to a number of the target chromosomes. When the number of the target chromosomes is 46, the target chromosome karyotype image is classified into that the subject has the normal number of chromosomes. When the number of the target chromosomes is greater than or less than 46, the target chromosome karyotype image is classified into that the subject has the abnormal number of chromosomes. Preferably, the abnormal number of chromosomes can include target chromosomes of the subject being the haploid or the polyploid.

The target feature selecting module 580 is for analyzing the target chromosome karyotype images to obtain at least one target image eigenvalue. The at least one target image eigenvalue can include the chromosome size, the chromosome location or the chromosome shape.

The comparing module 590 is for analyzing the at least one target image eigenvalue by the chromosome abnormality detecting model to obtain a target image eigenvalue weight data to determine whether the subject has the chromosome structural abnormality or the chromosome mosaicism. Preferably, the chromosome structural abnormality can include the target chromosomes of the subject being a chromosome deletion, a ring chromosome, a chromosome translocation, a chromosome inversion or a chromosome duplication.

In addition, the program of the non-transitory machine readable medium 500 can further include an assessing module (not shown) for calculating a value-at-risk of the subject having the chromosome abnormality according to the target image eigenvalue weight data.

### Examples

### I. Reference database

The reference database used in the present disclosure is the retrospective delinking clinical data of the subjects collected by the China Medical University Hospital. This clinical trial program is approved by China Medical University & Hospital Research Ethics Committee, which is numbered as CMUH107-REC3-151. The reference database includes reference metaphase chromosomes images of 30,000 reference subjects. The gender of the subjects of the metaphase chromosomes images is not particularly limited, and there is no special interval for the age of the subjects.

### II. The chromosome abnormality detecting model of the present disclosure

After obtaining the reference database, 23 pairs of chromosomes of each of the reference metaphase chromosomes images are arranged by the unsupervised learning classifier in the reference image transforming module to obtain a plurality of reference chromosome karyotype images.

In detail, the current deep neural network model requires a large amount of training data (that are the reference metaphase chromosomes images of the chromosome abnormality detecting model of the present disclosure) to achieve stable convergence and high classification accuracy. If the number of training data is insufficient, the deep neural network will be overfitting and the error value of the determination result will be too high, which makes the depth neural network model less reliable. In order to solve the problem, the chromosome abnormality detecting model of the present disclosure can further include an image preprocessing step, which is for correcting the black-and-white contrast on each of the reference chromosome karyotype images and normalizing the image values to obtain the image value interval between 0 and 1.

The preliminary classifying step is first performed to determine whether the reference subject has the abnormal number of chromosomes, which is classified according to the number of chromosomes in the reference chromosome karyotype image. When the number of chromosomes is 46, the reference chromosome karyotype image is classified into that a reference subject has the normal number of chromosomes. When the number of chromosomes is greater than or less than 46, the reference chromosome karyotype image is classified into that the reference subject the abnormal number of chromosomes.

Then, each of the reference chromosome karyotype images is analyzed by the feature selecting module to obtain at least one image eigenvalue. In detail, the feature selecting module can further distinguish the at least one image eigenvalue including the chromosome size, the chromosome location or the chromosome shape in each of the reference chromosome karyotype images.

Next, the at least one image eigenvalue is trained by the convolutional neural network learning classifier to achieve convergence, so as to obtain the chromosome abnormality detecting model of the present disclosure. In this example, the chromosome abnormality detecting model can be used to determine whether the subject has the abnormal number of chromosomes, the chromosome structural abnormality or the chromosome mosaicism. The convolutional neural network learning classifier can be the Inception-ResNet-v2 convolutional neural network or the Inception V3 convolutional neural network.

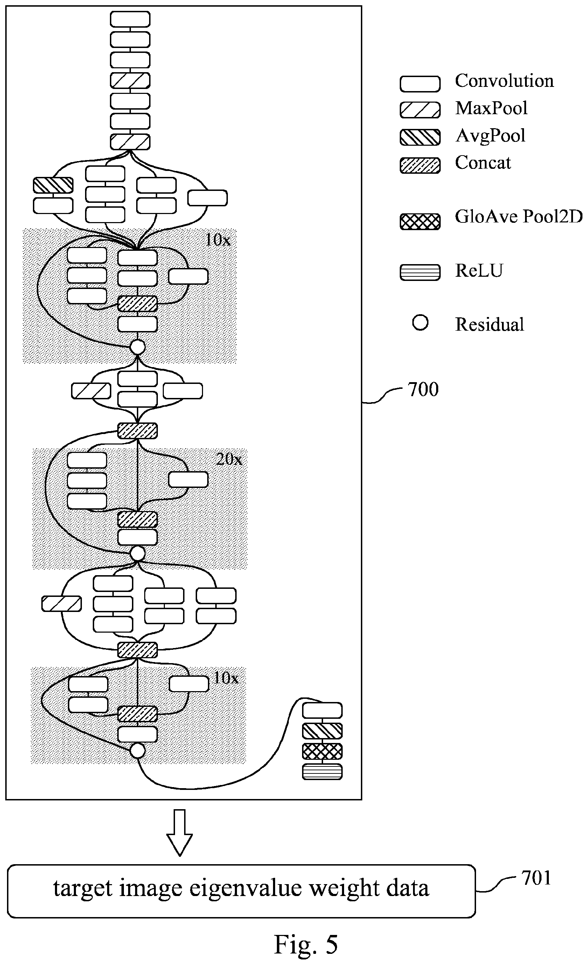

Please refer to Fig. 5, which is a structural diagram of a convolutional neural network learning classifier 700 of a chromosome abnormality detecting model according to one example of one embodiment of the present disclosure. In the example of Fig. 5, the convolutional neural network learning classifier 700 is the Inception-ResNet-v2 convolutional neural network, which includes a plurality of Convolution layers, a plurality of MaxPool layers, a plurality of AvgPool layers, and a plurality of Concat layers for training and analyzing the image eigenvalue.

In detail, the Inception-ResNet-v2 convolutional neural network is a large-scale visual recognition convolutional neural network based on the ImageNet visualization data database, and the image data in the ImageNet visualization data database are two-dimensional color images. Thus, the first convolutional layer of the conventional GoogLeNet convolutional neural network model has RGB three-channel filters. However, the original image files of each of the reference chromosome karyotype images are three-dimensional gray-scale images. The chromosome abnormality detecting model of the present disclosure further converts the GoogLeNet convolutional neural network model including the RGB three-channel filter into a single channel by arithmetic averaging, and applies the Stochastic Gradient Descent (SGD) to the pre-trained model neural network of the chromosome abnormality detecting model of the present disclosure for optimizing the training process thereof. The training frequency can be 100 epochs, gradient descent method can use 96 Mini-Batch Size, and the modulation is changed by changing the initial learning rate, wherein the learning rate is an important parameter for controlling weight and bias changes when training neural networks. Therefore, the chromosome abnormality detecting model of the present disclosure can further ensure that the loss function can reach stable convergence by adjusting the value of the learning rate.

In the process of training the image eigenvalue by the chromosome abnormality detecting model of the present disclosure, the image eigenvalue of each of the reference chromosome karyotype image is processed by two Convolution layers and one MaxPool layer to maximally output the extracted image eigenvalue. After repeating the two Convolutional layer and one MaxPool layer output, a plurality of Convolutional layers are used for parallel towers training to complete the inception training of the image eigenvalue.

After completing the inception training, the image eigenvalue of each of the reference chromosome karyotype image will be 10x, 20x and 10x Residual module trains at different depths, different layers and different aspects to train and converge the image eigenvalue of each of the reference chromosome karyotype image. In detail, since the Inception-ResNet convolutional neural network is subjected to a plurality of hierarchical weight operations, each residual module performs different operations and determinations on the image eigenvalues of each of the r reference chromosome karyotype image, resulting in error accumulation. Therefore, the training of the Inception-ResNet convolutional neural network will pull the node operation value of a specific layer back to the input end of the hierarchy and operate again to prevent the convolutional neural network learning classifier 700 from the degradation phenomenon of the gradient disappears caused by performing multi-layers weighting operation the image eigenvalue, and to avoid the accumulation of errors leading to information loss. Accordingly, the training efficiency of the convolutional neural network learning classifier 700 can be effectively improved.

After completing the deep and repeated residual module training, the converging image eigenvalue is finally trained and processed with one Convolutional layer, one AvgPool layer, one Global Average Pooling 2D (GloAvePool2D) layer, and one Rectified Linear Unit (ReLU) layer for determining the chromosomal abnormality of the subject. The AvgPool layer can first calculate the image eigenvalue of the residual module training to obtain an average value of each image eigenvalue. The GloAvePool2D layer can perform regularization processing on the overall network architecture of the convolutional neural network learning classifier 700 to prevent the error value of the determination result of the convolutional neural network learning classifier 700 from being too high caused by overfitting phenomenon under low-error training mode. Finally, the ReLU layer can further activate the image eigenvalue after completion of the training, and outputs a target image eigenvalue weight data 701 for subsequent comparison and analysis. The ReLU layer can prevent the target image eigenvalue weight data 701 outputted by the chromosome abnormality detecting model from approaching zero or approaching infinity, so as to facilitate the subsequent comparing step, thereby improving accuracy of the chromosome abnormality detecting model of the present disclosure.

Then, the determination results of the chromosomal abnormality of the subject are further integrated into the reference database to optimize the chromosome abnormality detecting model of the present disclosure, thereby further improving the training effect and the determination accuracy of the chromosome abnormality detecting model of the present disclosure.

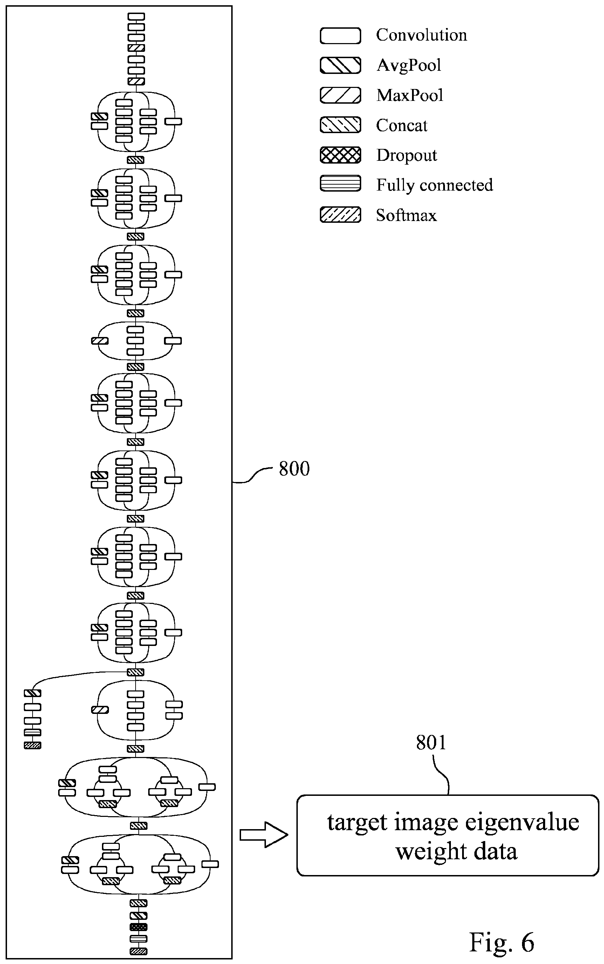

Please refer to Fig. 6, which is a structural diagram of a convolutional neural network learning classifier 800 of a chromosome abnormality detecting model according to another example of one embodiment of the present disclosure. In the example of Fig. 6, the convolutional neural network learning classifier 800 is the Inception V3 convolutional neural network, which includes a plurality of Convolution layers, a plurality of AvgPool layers, a plurality of MaxPool layers, and a plurality of Concat layers. The convolutional neural network learning classifier 800 further includes a Dropout layer, a Fully Connected layer, and a Softmax layer for solving the problem of overfitting on machine learning and for training and analyzing the image eigenvalue.

A single-layer neural network can cause problems of overfitting in machine learning because of too many parameters. The Inception V3 convolutional neural network is a factorization decomposition based on large filter size decomposition convolutional network. The Inception V3 convolutional neural network can reduce the order by parallel parameters, which can solve the problems of overfitting and increase the number of parameters by increasing the depth of the network to further approximate the mathematical model that is intended to be approximated.

In the process of training the image eigenvalue in the chromosome abnormality detecting model of the present disclosure, the image eigenvalue of each of reference chromosome karyotype image is respectively subjected to one AvgPool layer and one Convolutional layer, five Convolutional layers, three Convolutional layers, and one Convolutional layer for operation. After the operation, the values of the feature matrices of each group of operations are cascaded by the Concat layer. Then, the operations of one AvgPool layer and one Convolutional layer, five Convolutional layers, three Convolutional layers, and one Convolutional layer are respectively performed twice, and the values of the feature matrices of each group of operations are cascaded by the Concat layer. Next, operations of one MaxPool layer, three Convolutional layers, and one Convolutional layer are respectively performed, and the values of the feature matrices of each group of operations are cascaded by the Concat layer. Then, the operations of one AvgPool layer and one Convolutional layer, five Convolutional layers, three Convolutional layers, and one Convolutional layer are respectively performed 4 times, and the values of the feature matrices of each group of operations are cascaded by the Concat layer. Next, operations of one AvgPool layer, two Convolutional layers, one Fully Connected layer, and one Softmax layer are respectively performed. The value of the feature matrix of the operation is repeated twice to perform operations of one AvgPool layer and one Convolutional layer, three Convolutional layers and one Concat layer, two Convolutional layers and one Concat layer, and one Convolutional layer, and the values of the feature matrices of each group of operations are cascaded by the Concat layer. Finally, operations of one AvgPool layer, one Dropout layer, one Fully Connected layer, and one Softmax layer are performed, and a target image eigenvalue weighting data 801 is output to obtain a trained chromosome abnormality detecting model.

Then, the determination results of the chromosomal abnormality of the subject are further integrated into the reference database to optimize the chromosome abnormality detecting model of the present disclosure, thereby further improving the training effect and the determination accuracy of the chromosome abnormality detecting model of the present disclosure.

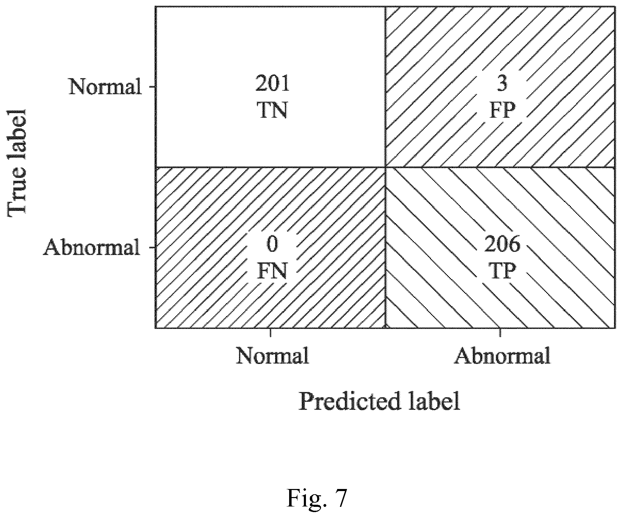

Please refer to Fig. 7, which is a confusion matrix of the chromosome abnormality detecting model of the present disclosure used to determine a chromosome abnormality of a subject. In the example of Fig. 7, the convolutional neural network learning classifier that establishes the chromosome abnormality detecting model is the convolutional neural network learning classifier 800 shown in Fig. 6 to determine whether the subject has the chromosome abnormality. The results are classified into normal and abnormal. In Fig. 7, the horizontal axis represents the prediction label, and the vertical axis represents the true label. The confusion matrix can be classified into True Positive (TP) block, True Negative (TN) block, False Positive (FP) block, and False Negative (FN) block. The correct rate, the sensitivity, the specificity, the positive predictive value and the negative predictive value of the chromosome abnormality detecting model of the present disclosure are calculated according to the number of subjects in the TP block (represented as "TP"), the number of subjects in the TN block (represented as "TN"), the number of subjects in the FP block (represented as "FP") and the number of subjects in the FN block (represented as "FN"). The correct rate is calculated by (TP + TN)/(TP + FP + TN + FN), the sensitivity is calculated by TP/(TP + FN), the specificity is calculated by TN/(TN + FP), the positive predictive value is calculated by TP/(TP + FP), and the negative predictive value is calculated by TN/(FN + TN).

In Fig. 7, the number of subjects in the TP block is 206, the number of subjects in the TN block is 201, the number of subjects in the FP block is 3, and the number of subjects in the FN block is 0. After calculation, the prediction result of the chromosome abnormality detecting model of the present disclosure for determining the chromosome abnormality of the subject is shown in Table 1.

**Table 1**

| | Prediction result (%) |
|---|---|
| Correct rate | 99.26 |
| Sensitivity | 100 |
| Specificity | 98.5 |
| Positive predictive value | 98.5 |
| Negative predictive value | 100 |

The results indicate that the chromosome abnormality detecting model of the present disclosure can be used accurately determine whether the subject has the chromosome abnormality, and the chromosome abnormality can include the abnormal number of chromosomes, the chromosome structural abnormality or the chromosome mosaicism.

Therefore, the chromosome abnormality detecting system and the method for detecting chromosome abnormality of the present disclosure can effectively improve the accuracy and sensitivity of the chromosome abnormality detection, and can shorten the evaluation time of the chromosome abnormality of the subject. From the original image input to the interpretation result, it takes only 0.1-1 seconds to complete, making the chromosome abnormality detecting system and the method for detecting chromosome abnormality of the present disclosure more widely used.

## Claims

1. A chromosome abnormality detecting model, comprising following establishing steps (100):
obtaining a reference database (110), wherein the reference database comprises a plurality of reference metaphase chromosomes images;
performing an image transforming step (120), wherein the image transforming step is for arranging 23 pairs of chromosomes of each of the reference metaphase chromosomes images by an unsupervised learning classifier to obtain a plurality of reference chromosome karyotype images;
performing a preliminary classifying step (130), wherein the preliminary classifying step is for classifying the reference chromosome karyotype images according to a number of the chromosomes, when the number of chromosomes is 46, the reference chromosome karyotype image is classified into that a reference subject has a normal number of chromosomes, and when the number of chromosomes is greater than or less than 46, the reference chromosome karyotype image is classified into that the reference subject has an abnormal number of chromosomes;
performing a feature selecting step (140), wherein the feature selecting step is for analyzing the reference chromosome karyotype images by using a feature selecting module to obtain at least one image eigenvalue; and
performing a training step (150), wherein the training step is for achieving a convergence of the image eigenvalue by using a convolutional neural network learning classifier (700, 800) to obtain the chromosome abnormality detecting model;
wherein the chromosome abnormality detecting model is used to determine whether a subject has a chromosome abnormality.

2. The chromosome abnormality detecting model of claim 1, wherein the unsupervised learning classifier is a Generative Adversarial Network (GAN).

3. The chromosome abnormality detecting model of claim 1 or claim 2, wherein the at least one image eigenvalue comprises a chromosome size, a chromosome location or a chromosome shape.

4. The chromosome abnormality detecting model of any one of claims 1 to 3, wherein the convolutional neural network learning classifier (700, 800) is an Inception-ResNet-v2 convolutional neural network or an Inception V3 convolutional neural network.

5. The chromosome abnormality detecting model of any one of claims 1 to 4, wherein the chromosome abnormality comprises an abnormal number of chromosomes, a chromosome structural abnormality or a chromosome mosaicism.

6. A method for detecting chromosome abnormality (200), comprising:
providing the chromosome abnormality detecting model of claim 1 (210);
providing a target metaphase chromosomes image of a subject (220);
arranging 23 pairs of chromosomes of the target metaphase chromosomes image by the unsupervised learning classifier to obtain a target chromosome karyotype image (230); and
using the chromosome abnormality detecting model to analyze the target chromosome karyotype image to determine whether the subject has a chromosome abnormality (240).

7. The method for detecting chromosome abnormality (200) of claim 6, wherein the chromosome abnormality comprises an abnormal number of chromosomes, a chromosome structural abnormality or a chromosome mosaicism.

8. The method for detecting chromosome abnormality (200) of claim 7, wherein the abnormal number of chromosomes comprises target chromosomes of the subject being a haploid or a polyploid.

9. The method for detecting chromosome abnormality (200) of claim 7, wherein the chromosome structural abnormality comprises target chromosomes of the subject being a chromosome deletion, a ring chromosome, a chromosome translocation, a chromosome inversion or a chromosome duplication.

10. A chromosome abnormality detecting system (300), comprising:
an image capturing unit (400) for obtaining a target metaphase chromosomes image (610) of a subject; and
a non-transitory machine readable medium (500) signal connected to the image capturing unit (400) and storing a program which, when executed by at least one processing unit, determines whether the subject has a chromosome abnormality, the program comprising:
a reference database obtaining module (510) for obtaining a reference database, wherein the reference database comprises a plurality of reference metaphase chromosomes images;
a reference image transforming module (520) for arranging 23 pairs of chromosomes of each of the reference metaphase chromosomes images by an unsupervised learning classifier to obtain a plurality of reference chromosome karyotype images;
a reference preliminary classifying module (530) for classifying the reference chromosome karyotype images according to a number of the reference chromosomes, when the number of the reference chromosomes is 46, the reference chromosome karyotype image is classified into that a reference subject has a normal number of chromosomes, and when the number of the reference chromosomes is greater than or less than 46, the reference chromosome karyotype image is classified into that the reference subject has an abnormal number of chromosomes;
a reference feature selecting module (540) for analyzing the reference chromosome karyotype images to obtain at least one reference image eigenvalue;
a training module (550) for achieving a convergence of the reference image eigenvalue by using a convolutional neural network learning classifier (700, 800) to obtain a chromosome abnormality detecting model;
a target image transforming module (560) for arranging 23 pairs of chromosomes of the target metaphase chromosomes image (610) by the unsupervised learning classifier to obtain a target chromosome karyotype image (620);
a target preliminary classifying module (570) for classifying the target chromosome karyotype images (620) according to a number of the target chromosomes, when the number of the target chromosomes is 46, the target chromosome karyotype image is classified into that the subject has the normal number of chromosomes, and when the number of the target chromosomes is greater than or less than 46, the target chromosome karyotype image is classified into that the subject has the abnormal number of chromosomes;
a target feature selecting module (580) for analyzing the target chromosome karyotype images (620) to obtain at least one target image eigenvalue; and
a comparing module (590) for analyzing the at least one target image eigenvalue by the chromosome abnormality detecting model to obtain a target image eigenvalue weight data (701, 801) to determine whether the subject has a chromosome structural abnormality or a chromosome mosaicism.

11. The chromosome abnormality detecting system (300) of claim 10, wherein the unsupervised learning classifier is a Generative Adversarial Network (GAN) .

12. The chromosome abnormality detecting system (300) of claim 10 or claim 11, wherein the at least one reference image eigenvalue comprises a chromosome size, a chromosome location or a chromosome shape, and the at least one target image eigenvalue comprises a chromosome size, a chromosome location or a chromosome shape.

13. The chromosome abnormality detecting system (300) of any one of claims 10 to 12, wherein the convolutional neural network learning classifier (700, 800) is an Inception-ResNet-v2 convolutional neural network or an Inception V3 convolutional neural network.

14. The chromosome abnormality detecting system (300) of any one of claims 10 to 13, wherein the program of the non-transitory machine readable medium (500) further comprises an assessing module for calculating a value-at-risk of the subject having the chromosome abnormality according to the target image eigenvalue weight data (701, 801).
